# EUROPEAN PATENT APPLICATION

(11) **EP 4 428 243 A1**
(43) Date of publication of application: **11.09.2024**
(21) Application number: 21963197.5
(22) Date of filing: 02.11.2021
(51) Int. Cl.: C12Q 1/60, C12Q 1/26

(54) **REAGENT COMPOSITION AND KIT**

(71) Applicant: Denka Company Limited, Tokyo 103-8338 (JP)
(72) Inventor: ITO, Yasuki, Tokyo 103-8338 (JP)
(74) Representative: Schnappauf, Georg
(86) International application number: PCT/JP2021/040433
(87) International publication number: WO 2023/079599

(57) **Abstract**

Provided is a reagent composition containing an anionic surfactant, in which the reagent composition is used as a first reagent composition in a method for quantifying a sdLDL-C in a sample, the method including acting the first reagent composition on a sample, and subsequently acting a second reagent composition for quantifying the sdLDL-C to quantify cholesterol in a remaining lipoprotein, the reagent composition has cholesterol esterase activity and cholesterol oxidase activity, and also has at least one activity selected from the group consisting of peroxidase activity and catalase activity, and a contact angle between the reagent composition and a polyethylene terephthalate (PET) base material is equal to or more than 65°and equal to or less than 77°.

## Description

### TECHNICAL FIELD

The present invention relates to a reagent composition and a kit.

### BACKGROUND ART

As a technique relating to a method of measuring LDL cholesterol, there is a method disclosed in Patent Document 1 (Japanese Unexamined Patent Publication No. 2000-325097). Patent Document 1 discloses a method for measuring lipoprotein cholesterol, which includes a first step of selectively subjecting HDL cholesterol to an enzyme reaction by adding an enzyme and a first surfactant to a sample containing a lipoprotein, a second step of selectively subjecting LDL cholesterol to an enzyme reaction by adding a second surfactant to the sample, and measuring a compound consumed by a reaction with the enzyme or a compound generated by the reaction in the first or second step to measure HDL cholesterol and/or LDL cholesterol. The method does not require a lipoprotein coagulant which increases a turbidity of a reaction solution, is not limited to the enzyme used, does not require an addition of another enzyme in the step of reacting the LDL cholesterol, can quantitatively measure the LDL cholesterol easily and inexpensively, and can accurately and inexpensively measure the HDL cholesterol without forming a lipoprotein coagulation which interferes with optical measurement. Therefore, the method is particularly useful in the field of clinical tests such as arteriosclerosis.

### RELATED DOCUMENT

### PATENT DOCUMENT

[Patent Document 1] Japanese Unexamined Patent Publication No. 2000-325097

### SUMMARY OF THE INVENTION

### TECHNICAL PROBLEM

The present inventor has investigated a measurement of small dense LDL cholesterol (sdLDL-C) among LDL cholesterols. As a result, it has been found that there is room for improvement in terms of measurement accuracy in the technique described in Patent Document 1.

The present invention provides a technique for measuring sdLDL-C with excellent accuracy.

### SOLUTION TO PROBLEM

The present inventor has conducted investigations to improve the accuracy of quantification of sdLDL-C. As a result, the present inventor has newly found that it is possible to quantify sdLDL-C with excellent accuracy by setting a contact angle of a reagent composition used for quantifying the sdLDL-C within a specific range, and has completed the present invention.

According to the present invention, the following reagent composition and kit are provided.
[1] A reagent composition containing:
   an anionic surfactant,
   in which the reagent composition is used as a first reagent composition in a method for quantifying a small dense LDL cholesterol (sdLDL-C) in a sample, the method including,
   acting the first reagent composition on the sample, and
   after the acting the first reagent composition on the sample, acting a second reagent composition for quantifying the sdLDL-C to quantify cholesterol in a remaining lipoprotein,
   the reagent composition has cholesterol esterase activity and cholesterol oxidase activity, and also has at least one activity selected from the group consisting of peroxidase activity and catalase activity, and
   a contact angle between the reagent composition and a polyethylene terephthalate (PET) base material, which is measured by the following method 1, is equal to or more than 65°and equal to or more than 77°,
   (method 1)
      (1) the PET base material: PET (amorphous polyester) resin plate, transparent, thickness of 2 mm,
      (2) a pre-treatment: a surface of the PET base material unused is wiped with a 70% ethanol aqueous solution, and a measurement is performed within 5 minutes after the wiping,
      (3) a measurement method and conditions: a liquid droplet method, a Θ/2 method, a temperature: 15 to 25°C, a syringe: Teflon (registered trademark; the same applies hereinafter)-coated 18G, a dropwise addition method, a dropwise addition amount: 2 µL, a measurement 1 second after the dropwise addition, and
      (4) a calculation of the contact angle: the measurement is performed 5 times, and an average value of the 5 times is obtained.
[2] The reagent composition according to [1], in which the reagent composition further has ascorbic acid oxidase activity.
[3] The reagent composition according to [1] or [2], in which the reagent composition has no sphingomyelinase activity.
[4] The reagent composition according to any one of [1] to [3], in which the anionic surfactant includes a sulfuric acid ester salt.
[5] The reagent composition according to any one of [1] to [4], further containing: any one of a hydrogen donor and a coupler.
[6] The reagent composition according to any one of [1] to [5], in which a viscosity of the reagent composition at 5°C, which is measured by the following method 2-1, is equal to or less than 2.0 mPa·s,
   (method 2-1)
   (1) a device: an Electro Magnetically Spinning (EMS) viscometer,
   (2) a measurement method and conditions: a measurement type: an electromagnetic spinning method, a motor rotation speed: 1,000 rpm, a holding time: 600 seconds, a measurement temperature: 5°C, a sequence loop: 1 time, a sample: 500 µL, and
   (3) a calculation of the viscosity: the measurement is performed 5 times, and an average value of the 5 times is calculated.
[7] The reagent composition according to any one of [1] to [6], in which a viscosity of the reagent composition at 37°C, which is measured by the following method 2-2, is equal to or less than 0.89 mPa·s,
   (method 2-2)
   (1) a device: an Electro Magnetically Spinning (EMS) viscometer,
   (2) a measurement method and conditions: a measurement type: an electromagnetic spinning method, a motor rotation speed: 1,000 rpm, a holding time: 600 seconds, a measurement temperature: 37°C, a sequence loop: 1 time, a sample: 500 µL, and
   (3) a calculation of the viscosity: the measurement is performed 5 times, and an average value of the 5 times is calculated.
[8] A kit used for quantifying a sdLDL-C in a sample, the kit containing:
   a first reagent composition consisting of the reagent composition according to any one of [1] to [7]; and
   a second reagent composition for quantifying the sdLDL-C.
[9] The kit according to [8], in which the second reagent composition has peroxidase activity.
[10] The kit according to [8] or [9],
   in which the first reagent composition contains any one of a hydrogen donor and a coupler and does not contain the other, and
   the second reagent composition does not contain the one of the hydrogen donor and the coupler and contains the other.

### ADVANTAGEOUS EFFECTS OF INVENTION

According to the present invention, it is possible to provide a technique for measuring sdLDL-C with excellent accuracy.

### BRIEF DESCRIPTION OF THE DRAWINGS

[Fig. 1] Diagrams showing evaluation results of accuracy of measured values of sdLDL-C.
[Fig. 2] Diagrams showing evaluation results of accuracy of measured values of sdLDL-C.
[Fig. 3] Diagrams showing evaluation results of accuracy of measured values of sdLDL-C.
[Fig. 4] Diagrams showing evaluation results of accuracy of measured values of sdLDL-C.

### DESCRIPTION OF EMBODIMENTS

Hereinafter, embodiments will be described. In the present embodiment, a composition such as a measurement reagent may contain each component alone or in combination of two or more thereof. In addition, in the present specification, a numerical range "x to y" indicates "x or more and y or less", and includes both the lower limit value x and the upper limit value y.

First, a lipoprotein will be described.

A lipoprotein can be fractionated roughly into Very Low Density Lipoprotein (VLDL), Low Density Lipoprotein (LDL) and High Density Lipoprotein (HDL), and LDL is sub-fractionated into a small dense LDL (sdLDL), and other sub-fractions. sdLDL is also referred to as small particle LDL, small LDL (SLDL), dense LDL, small, dense LDL, and other LDLs are sometimes referred to as large LDL (L LDL) or light LDL.

These lipoprotein fractions and sub-fractions may be distinguished based on a particle size or density.

The particle size of the lipoprotein in diameter is, 30 nm to 80 nm (or 30 nm to 75 nm) for VLDL, 22 nm to 28 nm (or 19 nm to 30 nm) for LDL, and 7 nm to 10 nm for HDL, although the figures may vary depending on the researchers.

The density of the lipoprotein is, for example, 1.006 or less for VLDL, 1.019 to 1.063 for LDL, and 1.063 to 1.21 for HDL.

Among lipoproteins, the diameter of LDL particles can be measured by, for example, gradient gel electrophoresis (GGE) (JAMA, 260, pp. 1917 to 1921, 1988) or NMR (HANDBOOK OF LIPOPROTEIN TESTING 2nd Edition, Edited by Nader Rifai et al. pp. 609 to 623, AACC PRESS: The Fats of Life Summer 2002, LVDD 15 YEAR ANNIVERSARY ISSUE, Volume AVI No. 3, pp. 15 to 16). The density can be determined based on, for example, analysis by ultracentrifugation (Atherosclerosis, 106, pp. 241 to 253, 1994: Atherosclerosis, 83, p. 59, 1990).

In the present embodiment, the sdLDL to be measured generally refers to a sub-fraction having a diameter of approximately 22.0 to approximately 25.5 nm or a sub-fraction having a density of 1.040 to 1.063 among LDL fractions.

The reason why LDL is sub-fractionated according to the particle size is that a small LDL among LDLs needs to be fractionally measured since LDL with a small particle diameter causes more arteriosclerosis and is higher in malignancy than other LDLs. Since the distributions of diameter and density of a LDL are continuous, it is not possible to determine the value of density above which the malignancy is clearly higher. Therefore, the density value of 1.040 to 1.063 described above is not an established characteristic of sdLDL, but is a value on the high density side obtained by dividing the density range of 1.019 to 1.063, which is widely used and established as the density of LDL, at a median point. For example, regarding the density of sdLDL, in a different report, sdLDL is fractionated in a range of 1.044 to 1.060 (Atherosclerosis: 106 241-253 1994). There are some differences among researchers on how to set the range of the density of sdLDL, but with any of the ranges chosen, the presence of sdLDL is associated with clinical malignancy.

In the present specification, in a case where sdLDL is referred to, it specifically refers to an LDL that has a higher density among LDLs and is clinically more arteriosclerogenic than other LDLs. In addition, sdLDL preferably has a higher density than the median point within the range of density for LDLs and more preferably refers to an LDL with the density in a range of 1.044 to 1.063. Furthermore, lipoproteins other than LDL refer to VLDL or HDL, and include chylomicrons, Intermediate Density Lipoproteins (IDL), or Very High Density Lipoproteins (VHDL).

### (Reagent composition (first reagent composition))

In the present embodiment, the reagent composition is used as a first reagent composition in a method for quantifying a small dense LDL cholesterol (sdLDL-C) in a sample, the method includes acting the first reagent composition on the sample, and after the acting the first reagent composition on the sample, acting a second reagent composition for quantifying the sdLDL-C to quantify cholesterol in a remaining lipoprotein.

Hereinafter, the reagent composition used as the first reagent composition is also simply referred to as a "first reagent composition".

The first reagent composition contains an anionic surfactant, has cholesterol esterase activity and cholesterol oxidase activity, and also has at least one activity selected from the group consisting of peroxidase activity and catalase activity. A contact angle between the first reagent composition and a polyethylene terephthalate (PET) base material, which is measured by the following method 1, is equal to or more than 65°and equal to or more than 77°.

### (Method 1)

(1) PET base material: PET (amorphous polyester) resin plate, transparent, thickness of 2 mm
(2) Pre-treatment: a surface of the PET base material unused is wiped with a 70% ethanol aqueous solution, and a measurement is performed within 5 minutes after the wiping
(3) Measurement method and conditions: a liquid droplet method, a Θ/2 method, a temperature: 15 to 25°C, a syringe: Teflon-coated 18G, a dropwise addition method, a dropwise addition amount: 2 µL, a measurement 1 second after the dropwise addition
(4) Calculation of the contact angle: the measurement is performed 5 times, and an average value of the 5 times is obtained

In the above (2), dust or the like on the PET base material is wiped off before the measurement. Since electrostatics affects the contact angle, the PET substrate is wiped off as much as possible so that friction or the like does not occur, and then the measurement is performed within 5 minutes.

In the present embodiment, the contact angle is specifically a static contact angle measured by, for example, DMo-901, 701, 601, 501, DMC-MC3, or the like manufactured by Kyowa Interface Science Co., Ltd.

Among measurement reagents for sdLDL-C, the present inventor has newly found that, by setting refractive indices of the first reagent composition and the PET base material in a specific range, it is possible to accurately quantify the sdLDL-C with excellent accuracy. The reason for this is not always clear, but the following is considered. In the quantification of sdLDL-C, for example, the first reagent composition is set in a biochemical automatic analyzer in a state of being put in a reagent bottle (such as PET), and is measured. At the time of measurement, a reagent probe sucks a certain amount of the first reagent composition and discharges the first reagent composition into a reaction cell. Since separability of the reagent liquid can be appropriately controlled by controlling the contact angle (wettability) of the first reagent composition used in eliminating cholesterol in lipoproteins other than sdLDL, the adsorption of the reagent component to the reagent bottle, the discharge from the probe, the adsorption in the reaction cell, and the like can be controlled, and as a result, the sdLDL-C can be measured more accurately.

From the viewpoint of further improving the accuracy of the sdLDL-C measurement, the contact angle between the reagent composition and the PET base material is 65° or more, preferably 66° or more and more preferably 67° or more.

In addition, from the viewpoint of further improving the accuracy of the sdLDL-C measurement, the contact angle between the reagent composition and the PET base material is 77° or less, preferably 76° or less and more preferably 75° or less.

In the present embodiment, for example, the contact angle with the PET base material can be controlled by appropriately selecting the type, combination, blending amount, and the like of each component contained in the first reagent composition. Among these, for example, the type and blending amount of a surfactant, the type and blending amount of a protein, and the like are exemplified as factors for setting the contact angle with the PET base material in a desired numerical range.

Since the first reagent composition is a composition containing an anionic surfactant and having at least one activity selected from the group consisting of cholesterol esterase activity and cholesterol oxidase activity, in a case where the first reagent composition is added to the sample, for example, it is possible to act on and eliminate lipoproteins other than sdLDL in the sample. In addition, cholesterol in the lipoproteins other than sdLDL can be led to the outside of the reaction system.

Here, the expression "surfactant acts on (reacts with)" refers to that the surfactant degrades lipoproteins to liberate cholesterol in the lipoproteins. For example, in a case of "surfactant which acts on (reacts with) the lipoproteins other than sdLDL", it is not required that the surfactant does not act on sdLDL at all, and the surfactant may mainly act on the lipoproteins other than sdLDL. The term "elimination" means that a substance in a test sample is degraded so as to prevent a degraded product thereof from being detected in the subsequent step. That is, the expression "eliminate cholesterol in lipoproteins other than sdLDL" means that the lipoproteins other than sdLDL in the test sample are degraded so as to prevent the cholesterol in the lipoproteins, which is a degraded product, from being detected in the subsequent step.

Specifically, the expression "lead to the outside of the reaction system" means that cholesterol contained in HDL, VLDL, L LDL, and the like is eliminated or aggregated so as to prevent the cholesterol contained in HDL, VLDL, L LDL, and the like from affecting the quantification of sdLDL-C, or is inhibited so as to avoid the reaction thereof in the subsequent step.

In addition, the expression "has cholesterol esterase activity" specifically means that cholesterol esterase is present and a reaction catalyzed by the cholesterol esterase can occur. The same applies to other enzyme activities such as the cholesterol oxidase activity.

Hereinafter, components contained in the first reagent composition will be described in more detail.

### (Anionic surfactant)

The first reagent composition contains at least an anionic surfactant. The anionic surfactant is specifically a surfactant which acts on the lipoproteins other than sdLDL, and more preferably a surfactant which acts on the lipoproteins other than sdLDL and does not act on the sdLDL.

Specific examples of the anionic surfactant include a sulfate-type anionic surfactant, a sulfonate-type anionic surfactant, a phosphate-type anionic surfactant, a carboxylic acid-type anionic surfactant, and an amino acid-based surfactant.

From the viewpoint of further improving the accuracy of the sdLDL-C measurement, the anionic surfactant preferably includes a sulfate-type anionic surfactant and more preferably includes a sulfuric acid ester salt. Specifically, the sulfuric acid ester salt is one or two or more kinds selected from the group consisting of an alkyl sulfuric acid ester salt, a polyoxyalkylene alkyl ether sulfuric acid ester salt, an amide ether sulfate, a polyoxyethylene alkyl phenyl ether sulfuric acid ester salt, a polyoxyethylene polycyclic phenyl ether sulfuric acid ester salt, a polyoxyethylene fatty acid amide sulfuric acid ester salt, and a polyoxyethylene alkylamine sulfuric acid ester salt, preferably one or two or more kinds selected from the group consisting of a polyoxyethylene alkyl phenyl ether sulfuric acid ester salt and a polyoxyethylene polycyclic phenyl ether sulfuric acid ester salt and more preferably one or two or more kinds selected from a polyoxyethylene polycyclic phenyl ether sulfuric acid ester salt.

Regarding other anionic surfactants, the sulfate-type anionic surfactant is specifically one or two or more kinds selected from the group consisting of an alkylbenzene sulfonate and an alkylnaphthalene sulfonate.

In addition, the amino acid-based surfactant is specifically an N-acylamino acid salt-type anionic surfactant such as an alkyl taurate.

In addition, in the anionic surfactant, specific examples of a cation constituting the salt include alkali metal ions such as a sodium ion and a potassium ion;
alkaline earth metal ions such as a magnesium ion and a calcium ion; and
ammonium.

From the viewpoint of further improving the accuracy of the sdLDL-C measurement, the cation constituting the salt is preferably at least one selected from the group consisting of an alkali metal ion and an ammonium, more preferably an alkali metal ion, and still more preferably a sodium ion.

From the viewpoint of stably acting the anionic surfactant on the lipoproteins other than sdLDL, a concentration of the anionic surfactant in the first reagent composition is preferably 0.005% (w/v) or more, more preferably 0.007% (w/v) or more, still more preferably 0.009% (w/v) or more, and even more preferably 0.010% (w/v) or more with respect to the total formulation of the first reagent composition.

In addition, the concentration of the anionic surfactant in the first reagent composition is preferably 0.05% (w/v) or less, more preferably 0.045% (w/v) or less, still more preferably 0.04% (w/v) or less, even more preferably 0.035% (w/v) or less, even still more preferably 0.03% (w/v) or less, and further more preferably 0.028% (w/v) or less with respect to the total formulation of the first reagent composition.

The anionic surfactant in the first reagent composition can be identified by, for example, a method of analyzing in combination with IR, NMR, LC-MS, and the like. Examples of a method for determining a structure of the anionic surfactant in the first reagent composition include a method of analyzing the anionic surfactant using LC-MSMS and NMR.

### (Enzyme)

The first reagent composition has cholesterol esterase (CHE) activity and cholesterol oxidase (COO) activity, and also has at least one activity selected from the group consisting of peroxidase (POD) activity and catalase activity. As a result, the first reagent composition can be stably acted on by the lipoproteins other than sdLDL to lead the cholesterol to the outside of the reaction system.

In addition, the first reagent composition specifically contains an enzyme having cholesterol esterase activity and an enzyme having cholesterol oxidase activity, and further contains at least one enzyme selected from the group consisting of an enzyme having peroxidase activity and an enzyme having catalase activity. More specifically, the first reagent composition contains cholesterol esterase, cholesterol oxidase, and at least one of peroxidase or catalase, preferably contains cholesterol esterase, cholesterol oxidase, and peroxidase.

As the cholesterol esterase, the cholesterol oxidase, the peroxidase, and the catalase, it is possible to use, for example, those derived from bacteria or fungi, or those derived from plants.

From the viewpoint of more stably leading cholesterol in the lipoproteins other sdLDL to the outside of the reaction system, the cholesterol esterase activity of the first reagent composition is preferably 50 U/L or more and more preferably 100 U/L or more, and is preferably 400 U/L or less, more preferably 350 U/L or less, and still more preferably 300 U/L or less; and it may be, for example, 150 U/L or less or 180 U/L or less.

From the same viewpoint, the cholesterol oxidase activity of the first reagent composition is preferably 100 U/L or more and more preferably 150 U/L or more, and is preferably 800 U/L or less, more preferably 750 U/L or less, and still more preferably 700 U/L or less; and it may be, for example, 200 U/L or less or 250 U/L or less.

From the viewpoint of more stably leading cholesterol in the lipoproteins other sdLDL to the outside of the reaction system, the peroxidase activity of the first reagent composition is preferably 200 U/L or more and more preferably 300 U/L or more, is preferably 3,000 U/L or less and more preferably 2,500 U/L or less; and it is also preferably, for example, 2,000 U/L or less. In addition, the peroxidase activity of the first reagent composition may be, for example, 5,000 U/L or less or 4,000 U/L or less.

From the viewpoint of stably eliminating hydrogen peroxide generated in a case where the first reagent composition is applied to the sample, and the viewpoint of more stably leading cholesterol in the lipoproteins other sdLDL in the sample to the outside of the reaction system, the catalase activity of the first reagent composition is preferably 100 U/mL or more and more preferably 200 U/mL or more, and is preferably 1,000 U/mL or less and more preferably 800 U/mL or less.

The cholesterol oxidase activity, the cholesterol esterase activity, the peroxidase activity, and the catalase activity of the first reagent composition can be measured by, for example, the following methods.

In the measurement of the cholesterol oxidase activity, a 6 mM cholesterol solution (cholesterol is dissolved in isopropanol) is used as a substrate solution. A dilution solution (0.1 M phosphate buffer solution, Triton X100, pH: 7.0) is added so that the concentration of the measurement target is 2 to 4 U/mL, 3 mL of the dilution solution is heated at 37°C for 5 minutes, and 0.05 mL of the substrate solution is added thereto. Thereafter, the mixed solution is allowed to react at 37°C, and an amount of change in absorbance at a wavelength of 240 nm is measured. For example, after the reaction at 37°C, the amount of change in absorbance from 2 minutes to 7 minutes is measured, and the cholesterol oxidase activity is calculated. For example, in a case where the amount of change in absorbance is 3 U/L or more, it can be said that the measurement target has cholesterol oxidase activity, and more specifically, it can be said that cholesterol oxidase is contained.

In the measurement of the cholesterol esterase activity, a substrate (a solution of 0.04% cholesterol linolenic acid, 1% Triton X100, and 0.6% sodium cholate), a solution of 300 U/mL cholesterol oxidase, an enzyme dilution solution (a 20 mM phosphate buffer solution containing 0.5 mM EDTA·2Na, 2 mM MgCl₂, and 0.2% bovine serum albumin (BSA), pH: 7.5), and a reaction solution (0.06% 4-aminoantipyrine, 0.4% phenol, 7.5 KU/L peroxidase (POD)) are used. After mixing 1.75 mL of the reaction solution and 1.0 mL of the substrate solution, the mixed solution is heated at 37°C for 5 minutes, and a 0.1 mL of cholesterol oxidase solution is added thereto. After heating the mixed solution at 37°C for 2 minutes, 0.1 mL of the measurement target diluted with the dilution solution is added, the mixed solution is allowed to react at 37°C, and the amount of change in absorbance at a wavelength of 500 nm is measured. For example, after the reaction at 37°C, the amount of change in absorbance from 0 minutes to 3.5 minutes is measured, and the cholesterol esterase activity is calculated. When the amount of change in absorbance is, for example, 8 U/L or more, it can be said that the measurement target has cholesterol esterase activity, and more specifically, it can be said that cholesterol esterase is contained.

The peroxidase activity of the first reagent composition is measured, for example, by the following method. That is, a reaction solution 1 (1.5 mM HDAOS, 0.05% Triton X100, and 50 mM phosphate buffer solution; pH: 7.0) and a reaction solution 2 (5 mM 4-aminoantipyrine, 0.05% Triton X100, 1% hydrogen peroxide, and 50 mM phosphate buffer solution; pH: 7.0), and a dilution solution (50 mM phosphate buffer solution, pH: 7.0) are used. 0.3 mL of the reaction solution 1 and 0.08 mL of the measurement target diluted with the dilution solution are mixed, and the mixed solution is heated at 37°C for 5 minutes. Thereafter, 0.1 mL of the reaction solution 2 is added thereto, the mixture is allowed to react at 37°C, and the amount of change in absorbance at a major wavelength of 600 nm and a minor wavelength of 700 nm is measured. For example, after the reaction at 37°C, the amount of change in absorbance from 2 minutes to 5 minutes is measured, and the peroxidase activity is calculated. For example, in a case where the amount of change in absorbance is 10 U/L or more, it can be said that the measurement target has peroxidase activity, and more specifically, it can be said that peroxidase is contained.

The catalase activity of the first reagent composition is measured, for example, by the following method. That is, in the measurement of the catalase activity, a substrate (0.06% hydrogen peroxide and 50 mM phosphate buffer solution; pH: 7.0) is used. After pre-heating 2.0 mL of the substrate solution at 25°C, the substrate solution is mixed with 0.1 mL of the measurement target, and the amount of change in absorbance at 240 nm is measured. For example, after the reaction at 25°C, the amount of change in absorbance from 0 minutes to 3 minutes is measured, and the catalase activity is calculated. For example, in a case where the amount of change in absorbance is 50 U/L or more, it can be said that the measurement target has catalase activity, and more specifically, it can be said that catalase is contained.

The first reagent composition may further have other enzyme activities, and specifically, one or two or more enzyme activities selected from the group consisting of ascorbic acid oxidase activity and lipoprotein lipase (LPL) activity.

In addition, the first reagent composition may contain, for example, an enzyme having one or two or more enzyme activities selected from the group consisting of an enzyme having ascorbic acid oxidase activity and an enzyme having lipoprotein lipase activity, and specifically contains one or two or more enzymes selected from the group consisting of ascorbic acid oxidase and lipoprotein lipase.

From the viewpoint of avoiding influence of ascorbic acid coexisting in the specimen on measurement accuracy, it is preferable that the first reagent composition further has the ascorbic acid oxidase activity, and it is more preferable that the first reagent composition has the ascorbic acid oxidase activity.

From the same viewpoint, the first reagent composition preferably contains an enzyme having ascorbic acid oxidase activity, and more preferably contains ascorbic acid oxidase.

From the viewpoint of avoiding influence of ascorbic acid coexisting in the specimen on measurement accuracy, the ascorbic acid oxidase activity of the first reagent composition is preferably 0.1 U/mL or more and more preferably 0.2 U/mL or more, and is preferably 15 U/mL or less and more preferably 10 U/mL or less.

The ascorbic acid oxidase activity of the first reagent composition is measured, for example, by the following method. That is, a substrate (10 mM ascorbic acid solution, 0.1% methacrylic acid) is used in the measurement of ascorbic acid oxidase activity. 0.25 mL of the measurement target diluted with a buffer solution (3.0 mL of 100 mM dimethylglytylic acid with a pH of 6.5 and 10 mM tris-HCL buffer solution with a pH of 8.0) is mixed, the mixture is pre-heated at 37°C for 2 minutes, 0.075 mL of the substrate solution is added thereto, and the reaction is carried out at 37°C to measure the amount of change in absorbance at 245 nm. For example, after the reaction at 37°C, the amount of change in absorbance from 0 minutes to 3.5 minutes is measured, and the ascorbic acid oxidase activity is calculated. For example, in a case where the amount of change in absorbance is 10 U/L or more, it can be said that the measurement target has ascorbic acid oxidase activity, and more specifically, it can be said that ascorbic acid oxidase is contained.

From the viewpoint of preferably adjusting the effects on the various lipoproteins, the lipoprotein lipase activity of the first reagent composition is preferably 2 U/mL or more and more preferably 50 U/mL or more, and is preferably 300 U/mL or less and more preferably 200 U/mL or less.

The lipoprotein lipase activity of the first reagent composition is measured, for example, by the following method. That is, in the measurement of the lipoprotein lipase activity, an olive oil emulsion solution (a solution obtained by adding 5 mL of 5.0% Triton X100 to 5 g of olive oil and 2 mL of ethanol, subjecting the mixture to sonication for 20 minutes, subsequently adding 25 mL of 4% BSA and 15 mL of a 0.1 M phosphate buffer solution with a pH 7.0 thereto, and carrying out stirring at room temperature for 1 to 2 hours) is used as a substrate solution. A dilution solution (a 20 mM phosphate buffer solution containing 2 mM MgCl₂ and 0.5 mM EDTA-2Na; pH: 7.5) is added to the substrate solution pre-heated at 37°C for 5 minutes so that the concentration thereof is 0.9 to 1.6 U/mL, 0.2 mL of a diluted specimen is added thereto, and after heating at 37°C for 15 minutes, 2.0 mL of a reaction stopping solution (0.2 M trichloroacetic acid) is added thereto. Thereafter, filtration (ADVANTEC No. 131 of Toyo Roshi Kaisha, Ltd. or Whatman No. 42) is carried out to collect the filtrate. 3.0 mL of a color developing reagent (200 mL of a 50 mM MES-NaOH buffer solution, 4 mL of 5% Triton X100, 40 µL of N,N-dimethyl-m-toluidine, 4 mg of 4-aminoantipyrine, 24.2 mg of ATP·2Na·3H₂O, 40.7 mg of MgCl₂·6H₂O, 200 U of glycerol kinase, 500 U of Lα glycerophosphate oxidase, and 300 U of peroxidase) is added to 0.05 mL of the filtrate, the mixed solution is allowed to react at 37°C for 15 minutes, the absorbance at a wavelength of 545 nm is measured before and after the reaction, and the lipoprotein lipase activity is calculated. For example, in a case where the amount of change in absorbance is 3 U/L or more, it can be said that the measurement target has lipoprotein lipase activity, and more specifically, it can be said that lipoprotein lipase is contained.

The lipoprotein lipase is not limited as long as it is an enzyme capable of degrading a lipoprotein, and it is possible to use, for example, a lipoprotein lipase derived from an animal or a microorganism.

In addition, from the viewpoint that the first reagent composition does not excessively eliminate the sdLDL-C which is the measurement target in liberating cholesterol in the lipoproteins other than sdLDL and leading the cholesterol to the outside of the reaction system (first step described later), it is preferable that the first reagent composition does not have sphingomyelinase activity. From the same viewpoint, the first reagent composition preferably does not substantially contain an enzyme having sphingomyelinase activity, and more preferably does not substantially contain sphingomyelinase.

Here, the expression "does not have sphingomyelinase activity" specifically means that sphingomyelinase activity measured by the following method is less than 2 U/L, preferably 1 U/L or less, more preferably 0.1 U/L or less, and still more preferably 0 U/L.

In addition, the expression that the first reagent composition does not substantially contain the enzyme having sphingomyelinase activity and the sphingomyelinase means that these enzymes, which are identified by a method described later, are below the detection limit.

The sphingomyelinase activity of the first reagent composition is measured, for example, by the following method. That is, a reaction solution (a mixed solution of 0.008% sphingomyelin, a 0.05% Triton X100 solution, 10 U/mL alkaline phosphatase, 10 U/mL cholesterol oxidase, 2 U/mL peroxidase, 0.02% 4-aminoantipyrine, and 0.02% TODB), a reaction stopping solution (1% sodium dodecyl sulfate solution), and a dilution solution (10 mM Tris buffer solution containing 0.1% Triton X100; pH: 8.0) are used. 0.08 mL of the reaction solution and 0.003 mL of the measurement target diluted with the dilution solution are mixed, and after heating at 37°C for 5 minutes, 0.16 mL of the reaction stopping solution is added thereto. After stopping the reaction, the amount of change in absorbance at a major wavelength of 546 nm and a minor wavelength of 700 nm is measured to calculate the sphingomyelinase activity. For example, in a case where the amount of change in absorbance is 2 U/L or more, it can be said that the measurement target has sphingomyelinase activity, and more specifically, it can be said that sphingomyelinase is contained.

In addition, in the present embodiment, enzymes in the first reagent composition and enzymes in a second reagent composition described later can also be identified by the following method. That is, first, a fragment peptide obtained by degrading a sample containing a target enzyme with trypsin is detected by a hybrid mass spectrometer. Proteins can be identified by database search (for example, Mascot search) of the mass of peptides obtained using a mass spectrometer and the spectrum (MS/MS data) of fragment ions obtained by colliding with argon gas in the mass spectrometer. In a case where the sequence of the fragment peptide derived from the amino acid sequence in the reagent composition matches with the amino acid sequence registered in the database as a unique sequence, it can be considered that the target enzyme is contained.

In addition, the enzymes in the first reagent composition and the enzymes in the second reagent composition described later can be identified, for example, by the following quantification. That is, among the fragment peptides obtained by degrading the target enzyme with trypsin, a peptide that is specific to the target enzyme and gives a strong signal in mass spectrometry is selected as a peptide to be quantified. For the peptide to be quantified, an unlabeled peptide and a peptide labeled with a stable isotope as an internal standard are prepared by chemical synthesis. A sample containing the target enzyme is completely digested with trypsin, a known amount of stable isotope-labeled peptide is added, and the measurement is carried out with a triple quadrupole mass spectrometer (LC-MS/MS) connected to HPLC, in a multiple reaction monitoring mode (MRM mode). A mixed solution of the unlabeled peptide of the peptide to be quantified and the known amount of stable isotope-labeled peptide is measured in the same manner to create a calibration curve of the concentration ratio and the peak area ratio of the internal standard, and the absolute amount of the peptide to be quantified in the sample is calculated. As a result, the target enzyme can be quantified.

### (Other components)

The first reagent composition may contain a component other than the components described above. Examples of other components include a buffer solution, a salt, a protein having no enzymatic action, a preservative, a hydrogen donor, and a coupler.

The type of the buffer solution can be appropriately selected, for example. Specific examples of the buffer solution include a MOPS (3-morpholinopropane sulfonic acid) buffer solution, a phosphate buffer solution, a Tris buffer solution, a PIPES (piperazine-1,4-bis(2-ethanesulfonic acid)) buffer solution, and a HEPES (4-(2-hydroxyethyl)-1-piperazineethanesulfonic acid) buffer solution.

From the viewpoint of maintaining the enzyme activity in the composition and the viewpoint of improving storage stability of the reagent, a concentration of the buffer solution is preferably 1 mM or more, more preferably 5 mM or more, and still more preferably 10 mM or more, and is preferably 300 mM or less, more preferably 200 mM or less, still more preferably 150 mM or less, and even more preferably 100 mM or less.

Specifically, the salt is blended as a pH adjuster or an ionic strength adjuster. Specific examples of the salt include basic substances including sodium salts such as sodium hydroxide and sodium sulfate, potassium salts such as potassium hydroxide, and ammonium salts such as ammonium sulfate and ammonium chloride. In addition, in a case where the first reagent composition contains at least one of a monovalent cation and a divalent cation, or a salt thereof, the fractionation of sdLDL and L LDL becomes easier.

From the viewpoint of allowing the salt to act more stably as the pH adjuster or the ionic strength adjuster, a concentration of the salt in the first reagent composition is preferably 0.2 g/L or more and more preferably 0.5 g/L or more, and is preferably 5 g/L or less and more preferably 3 g/L or less, with respect to the entire formulation of the first reagent composition.

From the viewpoint of maintaining the enzyme activity in the composition and the viewpoint of improving storage stability of the reagent, a pH of the first reagent composition is preferably 6 or more and more preferably 6.5 or more, and is preferably 8 or less and more preferably 7.5 or less.

Specific examples of the protein having no enzymatic action include albumin such as BSA.

From the viewpoint of stabilizing the enzymes in the first reagent composition and the viewpoint of stabilizing the first step of leading the cholesterol in the lipoproteins other than sdLDL to the outside of the reaction system, a concentration of the albumin such as BSA in the first reagent composition is preferably 1 g/L or more and more preferably 2 g/L or more, and is preferably 20 g/L or less and more preferably 10 g/L or less, with respect to the total formulation of the first reagent composition.

The first reagent composition preferably contains any one of a hydrogen donor and a coupler. In this case, any one of the hydrogen donor and the coupler is used in the first step to lead the cholesterol in the lipoproteins other than sdLDL to the outside of the reaction system. More specifically, any one of the hydrogen donor and the coupler is used in order to cause the cholesterol esterase or the cholesterol oxidase to act on the cholesterol in the lipoproteins other than sdLDL and to convert the generated hydrogen peroxide to colorless quinone in the presence of the peroxidase.

Specific examples of the hydrogen donor include aniline derivatives such as N-ethyl-N-(2-hydroxy-3-sulfopropyl)-3-methylaniline (TOOS), N-ethyl-N-(2-hydroxy-3-sulfopropyl)-3,5-dimethylaniline (MAOS), N-ethyl-N-(3-sulfopropyl)-3-methylaniline (TOPS), N-(2-hydroxy-3-sulfopropyl)-3,5-dimethoxyaniline (HDAOS), N-(3-sulfopropyl)aniline (HALPS), N-(3-sulfopropyl)-3-methoxy-5-aniline (HMMPS), N-ethyl-N-(2-hydroxy-3-sulfopropyl)-4-fluoro-3,5-dimethoxyaniline (FDAOS), and N-ethyl-N-(3-methylphenyl)-N'-succinylethylenediamine (EMSE).

From the viewpoint of leading the cholesterol in the lipoproteins other than sdLDL to the outside of the reaction system, a concentration of the hydrogen donor in the first reagent composition is preferably 1 mM or more and more preferably 1.5 mM or more, and is preferably 5 mM or less and more preferably 3 mM or less.

In addition, in a case where the hydrogen donor is used in the second reagent composition described later, the coupler used in the coupling reaction is used in the first reagent composition. As the coupler, for example, 4-aminoantipyrine, an aminoantipyrine derivative, vanillindiamine sulfonic acid, methylbenzthiazolinone hydrazone, sulfonated methylbenzthiazolinone hydrazone, or the like can be used, but the coupler is not limited thereto.

Next, physical properties of the first reagent composition will be described.

A property of the first reagent composition is specifically liquid.

From the viewpoint of improving the accuracy of the measurement, a viscosity of the first reagent composition at 5°C may be, for example, 3.0 mPa·s or less, and preferably 2.0 mPa·s or less and more preferably 1.9 mPa·s or less.

In addition, the viscosity of the first reagent composition at 5°C may be, for example, 1.4 mPa·s or more, or may be, for example, 1.5 mPa·s or more.

From the viewpoint of improving the accuracy of the measurement, a viscosity of the first reagent composition at 37°C is preferably 0.89 mPa·s or less, more preferably 0.87 mPa·s or less, and still more preferably 0.85 mPa·s or less; and it is also preferably 0.80 mPa·s or less or 0.75 mPa or less.

In addition, the viscosity of the first reagent composition at 37°C may be, for example, 0.5 mPa·s or more.

Here, the viscosity of the first reagent composition at each temperature is measured by the following method 2. In the method 2, a method in which the measurement temperature is set to 5°C is a method 2-1, and a method in which the measurement temperature is set to 37°C is a method 2-2.

### (Method 2)

(1) Device: Electro Magnetically Spinning (EMS) viscometer
(2) Measurement method and conditions: a measurement type: an electromagnetic spinning method, a motor rotation speed: 1,000 rpm, a holding time: 600 seconds, a measurement temperature: 5°C (method 2-1) and 37°C (method 2-2), a sequence loop: 1 time, a sample: 500 µL
(3) Calculation of the viscosity: the measurement is performed 5 times, and an average value of the 5 times is calculated

In the present embodiment, the viscosity is measured by, for example, EMS-1000 manufactured by KYOTO ELECTRONICS MANUFACTURING CO., LTD.

In addition, among measurement reagents for sdLDL-C, the present inventor has newly found that, by setting the viscosity of the first reagent composition in a specific range, it is possible to quantify the sdLDL-C with more excellent accuracy. The reason for this is not always clear, but the following is considered. In the quantification of sdLDL-C, at the time of measurement, a reagent probe sucks a certain amount of the first reagent composition and discharges the first reagent composition into a reaction cell. In a case where the viscosity of the first reagent composition used in the eliminating the cholesterol in the lipoproteins other than sdLDL is too high, a discharge amount may change due to the viscosity, and the eliminated amount may fluctuate. However, since the separation of the reagent liquid can be appropriately controlled by accurately controlling the viscosity, it is considered that as a result, the sdLDL-C can be measured more accurately.

In the present embodiment, since the first reagent composition contains specific components and has a contact angle in a specific range, the first reagent composition can be suitably used for quantifying sdLDL-C and can have excellent accuracy in the measurement of sdLDL-C.

For example, the first reagent composition is used in combination with the second reagent composition described later for quantifying the sdLDL-C.

### (Kit)

In the present embodiment, the kit is used for quantifying sdLDL-C in a sample, and contains the above-described first reagent composition and a second reagent composition for quantifying sdLDL-C.

In addition, the kit is specifically used for a method of quantifying sdLDL-C, which includes two or more steps. At this time, the first and second reagent compositions are used in different steps, and it is preferable that the first reagent composition and the second reagent composition are used in this order.

Hereinafter, a configuration of the second reagent composition will be described in more detail.

### (Second reagent composition)

The second reagent composition is specifically a reagent composition for quantifying sdLDL-C. Although components of the second reagent composition differ depending on the configuration of the first reagent composition, any blending formulation capable of quantifying sdLDL-C may be used, and known substances can be used.

Specific examples of the components contained in the second reagent composition include an enzyme, a buffer solution, a salt, a surfactant, a protein having no enzymatic action, a preservative, and any one of a hydrogen donor and a coupler.

In addition, in a preferred configuration of the kit, the first reagent composition contains any one of a hydrogen donor and a coupler and does not contain the other, and the second reagent composition does not contain the one of the hydrogen donor and the coupler and contains the other.

Examples of the enzyme include peroxidase. In addition, for example, the second reagent composition has peroxidase activity. From the viewpoint of accurately measuring the sdLDL-C in the second step, the peroxidase activity of the second reagent composition is preferably 500 U/L or more and more preferably 1,000 U/L or more, and is preferably 10,000 U/L or less. Since the peroxidase activity is exhibited over the second step in a case where the first reagent composition has the peroxidase activity, it is also possible to reduce the concentration or make the peroxidase not contained in the second reagent.

The types of buffer solution and salt can be appropriately selected depending on, for example, the type of enzyme contained in the second reagent composition. Specific examples of the buffer solution include the buffer solutions described for the first reagent composition above.

From the viewpoint of maintaining the enzyme activity in the composition and the viewpoint of improving storage stability of the reagent, a pH of the second reagent composition is preferably 6 or more and more preferably 6.5 or more, and is preferably 8 or less and more preferably 7.5 or less.

Examples of the surfactant include surfactants which act on sdLDL. In addition, from the viewpoint of stably quantifying sdLDL-C, the second reagent composition preferably contains a surfactant which acts on sdLDL.

The surfactant which acts on sdLDL may be a surfactant which selectively acts on sdLDL, such as a surfactant which acts only on sdLDL, a surfactant which also acts on lipoproteins other than sdLDL, or a surfactant which acts on all lipoproteins.

As the surfactant in the second reagent composition, for example, a polyoxyethylene derivative may be mentioned, and any commercially available surfactant can be used as long as the surfactant is used in reagents or the like for total cholesterol measurement. Examples of such a surfactant include polyoxyethylene alkyl phenyl ether such as polyoxyethylene octylphenyl ether (for example, Emulgen 909 (manufactured by Kao Corporation), and Triton X-100) and polyoxyethylene alkyl ether (for example, Emulgen 707 and Emulgen 709 (manufactured by Kao Corporation)).

From the viewpoint of stably acting on sdLDL, a concentration of the surfactant in the second reagent composition is preferably 0.01% (w/v) or more, more preferably 0.02% (w/v) or more, and still more preferably 0.03% (w/v) or more with respect to the mixed solution after addition of the second reagent composition.

In addition, from the same viewpoint, the concentration of the surfactant in the second reagent composition is preferably 2.0% (w/v) or less and more preferably 1.0% (w/v) or less.

In a case where the second reagent composition contains a coupler, the coupler is preferably one or two or more compounds selected from the group consisting of 4-aminoantipyrine (4-AA), an aminoantipyrine derivative, vanillindiamine sulfonic acid, methylbenzthiazolinone hydrazone, and sulfonated methylbenzthiazolinone hydrazone.

From the viewpoint of stably acting on sdLDL, a concentration of the coupler in the second reagent composition is preferably 0.5 mM or more and more preferably 1.0 mM or more, and is preferably 15 mM or less and more preferably 10 mM or less, with respect to the total formulation of the reaction solution after addition of the second reagent composition.

On the other hand, in a case where the coupler is contained in the first reagent composition, the hydrogen donor is preferably contained in the second reagent composition. In this case, from the viewpoint of stably acting on sdLDL, a concentration of the hydrogen donor in the sample is preferably 3 mM or more and more preferably 4.5 mM or more, and is preferably 15 mM or less and more preferably 12 mM or less, with respect to the total formulation of the reaction solution after addition of the second reagent composition.

Specific examples of the protein having no enzymatic action include the proteins described for the first reagent composition above.

### (Method)

The method in the present embodiment is a method of quantifying sdLDL-C in a sample using the above-described first and second reagent compositions. The quantification method in the present embodiment includes the following first step and second step.
(First step) acting the above-described first reagent composition on the sample
(Second step) acting the above-described second reagent composition on the sample after the first step to quantify cholesterol in the remaining lipoprotein

The configurations of the first and second reagent compositions are as described above. In such a method, by using the first reagent composition according to the present embodiment, it is possible to perform the measurement with excellent accuracy. Therefore, for example, it is also possible to stably quantify the sdLDL-C with high accuracy.

In addition, the method of quantifying sdLDL-C may be performed by, for example, adding the first reagent composition to a sample (test sample) to allow a reaction to proceed, then adding the second reagent composition to the sample to allow a reaction to proceed, and measuring the absorbances thereof.

The test sample is, for example, a sample derived from blood such as serum and plasma, and preferably serum.

The first and second steps are usually performed in an automatic analyzer.

The amount of the sample and the amount of each reagent composition can be appropriately determined in consideration of, for example, the concentration of the reagent in each reagent composition, and the amounts are determined within a range applicable to the automatic analyzer. For example, it is sufficient that 1 to 10 µL of the test sample, 50 to 300 µL of the first reagent composition, and 25 to 200 µL of the second reagent composition are used.

Hereinafter, each step will be described in more detail.

### (First step)

In the first step, the first reagent composition is allowed to act on the sample. As a result, lipoproteins other than sdLDL are eliminated, and cholesterol in the lipoproteins other than sdLDL is liberated and led to the outside of the reaction system.

More specifically, in the first step, preferably, a surfactant which acts on lipoproteins other than sdLDL is allowed to act on the sample in the presence of cholesterol esterase. Thereafter, cholesterol generated by liberation from the lipoproteins is allowed to react with an enzyme which reacts with cholesterol, such as cholesterol oxidase, and is led to the outside of the reaction system. In the first step, for example, known techniques for eliminating cholesterol in the lipoproteins other than sdLDL and leading the cholesterol to the outside of the reaction system, aggregating the cholesterol in the lipoproteins other than sdLDL, inhibiting the cholesterol in the lipoproteins other than sdLDL to avoid the reaction in the subsequent step, and the like can be used.

In a case where the first reagent composition has an electron donor, the eliminating the cholesterol generated from the lipoproteins other than sdLDL and leading the cholesterol to the outside of the reaction system in the first step may include, for example, forming a colorless quinone in the presence of hydrogen peroxide generated by cholesterol esterase activity and cholesterol oxidase activity in the first reagent composition and of the electron donor.

In addition, in the first step, at least one of a monovalent cation and a divalent cation, or a salt thereof can be used as an ionic strength adjuster by further adding such an ionic strength adjuster to the reaction solution. Addition of such an ionic strength adjuster facilitates differentiation of sdLDL and L LDL.

### (Second step)

In the second step, the sdLDL-C remaining after the first step is quantified. In the second step, an LDL quantification method conventionally used can be used. Examples thereof include a method of quantifying a content of an LDL specific aggregate formed by adding an LDL aggregating agent according to turbidimetry, a method of using an antigen-antibody reaction with an LDL specific antibody, a method of quantifying a degraded product using an enzyme. The quantification method is selected, for example, according to the components contained in the second reagent composition or the formulation thereof.

In a case where the second reagent composition contains an enzyme, the method of quantifying a degraded product using an enzyme can be adopted. Specifically, the second reagent composition containing, for example, one or two or more enzymes for measuring cholesterol, selected from the group consisting of cholesterol esterase, cholesterol oxidase, cholesterol dehydrogenase and peroxidase, is added to the reaction solution after the first step, to liberate and degrade sdLDL-C and quantifying the reaction product thereof.

In the present embodiment, the reaction temperature in each step is preferably 2°C to 45°C, and more preferably 25°C to 40°C.

Regarding the reaction time, each step is preferably performed for 1 to 10 minutes and more preferably performed for 3 to 7 minutes.

Examples of the automatic analyzer used for quantifying sdLDL-C include TBA-120FR and TBA-200FR (all manufactured by TOSHIBA Corporation), JCA-BM 1250, JCA-BM 1650, and JCA-BM 2250 (all manufactured by JEOL Ltd.), HITACHI 7180, and HITACHI 7170 (all manufactured by Hitachi, Ltd.), AU2700, AU5800, and AU680 (all manufactured by Olympus Corporation), and cobas c501 and cobas c701 (all manufactured by Roche).

The sdLDL-C is quantified by measuring the absorbance, for example, in a wavelength range of 580 to 720 nm, preferably 600 to 700 nm.

The embodiments of the present invention have been described above, but these are examples of the present invention and various configurations other than the above can be adopted. Examples

### (Examples 1 to 7 and Comparative Examples 1 and 2)

A first reagent composition of each of Examples was prepared, and the accuracy of measurement in a case of being combined with a second reagent composition and used for quantifying sdLDL-C was evaluated.

### (Preparation of reagent composition)

The first reagent composition and the second reagent composition of each of Examples were prepared by blending each of the following components at the following concentrations.

Regarding the first reagent composition, the composition of each of Examples was prepared by changing the concentration of bovine serum albumin and the anionic surfactant.

The blending formulations of the first reagent composition and the second reagent composition used for the measurement are shown below.

### (First reagent composition)

| | |
|---|---|
| MOPS buffer solution (pH: 7.0) | 50 mM |
| Cholesterol esterase | 300 U/mL |
| Cholesterol oxidase | 600 U/mL |
| Peroxidase | 2,500 U/L |
| Bovine serum albumin | |
| TOOS | 2.0 mM |
| Ascorbic acid oxidase | 3.0 U/mL |

### Anionic surfactant (polyoxyethylene polycyclic phenyl ether sulfuric acid ester salt)

### (Second reagent composition)

| | |
|---|---|
| MOPS buffer solution (pH: 7.0) | 50 mM |
| 4-aminoantipyrine | 4.0 mM |
| Peroxidase | 4,000 U/L |
| Sodium azide | 0.05% (w/v) |
| Polyoxyethylene octylphenyl ether | 1% (w/v) |

### (Method for measuring contact angle)

As a PET base material, a visually transparent PET (amorphous polyester) resin plate (manufactured by TAKIRON Corporation, PET plate (PETEC) product number 6010, thickness: 2 mm) was used. A surface of the unused PET base material was wiped with a 70% ethanol aqueous solution, and then the measurement was performed within 5 minutes.

Using a contact angle meter (manufactured by Kyowa Interface Science Co., Ltd., DMo-501), the contact angle was measured 1 second after the dropwise addition under the conditions of a temperature of 15 to 25°C, a syringe of Teflon-coated 18 G, a dropwise addition method, and a dropwise addition amount of 2 µL by a liquid droplet method and a Θ/2 method.

The measurement was performed 5 times for each of Examples, and the average value of the 5 times was calculated. The measurement results are shown in Table 1 or Table 3.

### (Method for measuring viscosity)

For Examples 1 to 7, the viscosity of the first reagent composition in each of Examples was measured by an electromagnetic spinning method using an EMS viscometer (EMS-1000 manufactured by KYOTO ELECTRONICS MANUFACTURING CO., LTD.) at a motor rotation speed of 1,000 rpm, a holding time of 600 seconds, measurement temperatures of 5°C and 37°C, a sequence loop of 1 time, and a sample of 500 µL. For each of Examples, the measurement was performed 5 times at each temperature, and the average value of the 5 times was calculated. The measurement results are shown in Table 2 or Table 3.

### (Accuracy test)

The accuracy when measuring sdLDL-C having a known concentration using the first reagent composition of each of Examples was evaluated by a comparative reference method. Specifically, in each of Examples, that is, the first reagent composition having different contact angles and the second reagent composition were prepared. 75 µL of the first reagent composition was added to 2 µL of the serum sample, the mixture was reacted at 37°C for 5 minutes, and then 75 µL of the second reagent composition was added thereto and reacted for 5 minutes. Thereafter, an absorbance at a major wavelength of 600 nm and a minor wavelength of 700 nm was measured.

As the comparative reference method, the sdLDL cholesterol concentration was compared using a reagent for measuring sdLDL cholesterol, sdLDL-C "SEIKEN" manufactured by Denka Seiken Co., Ltd.

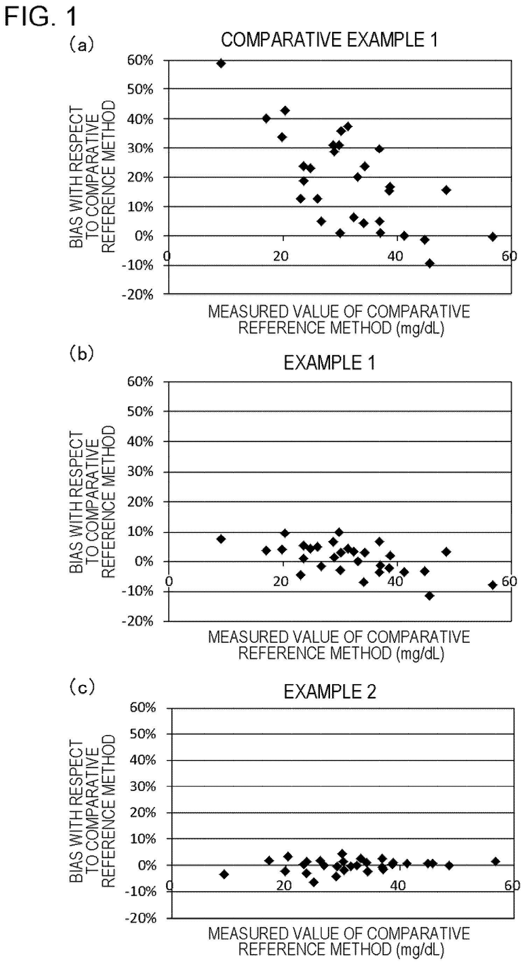

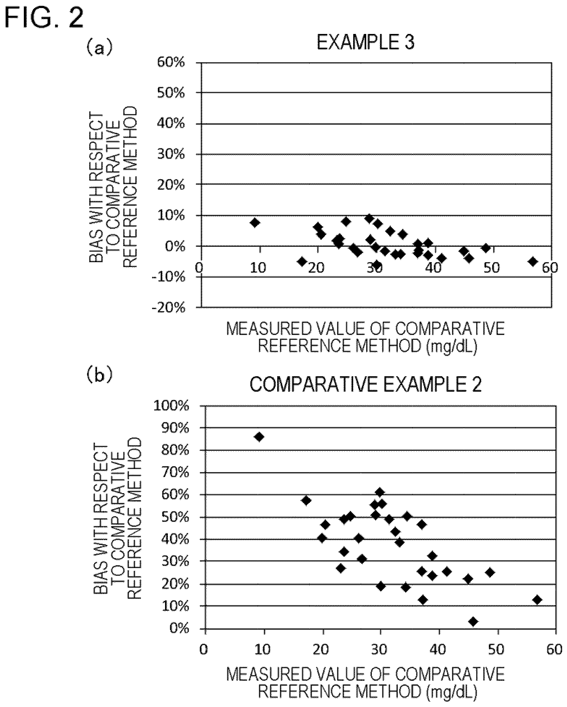

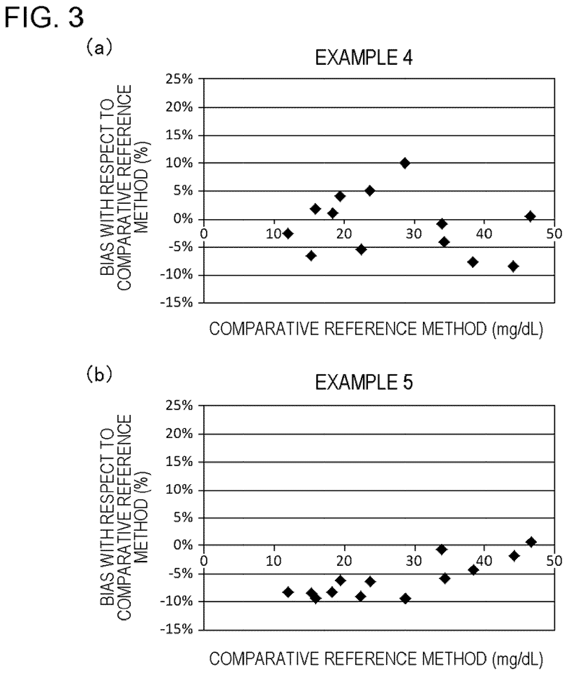

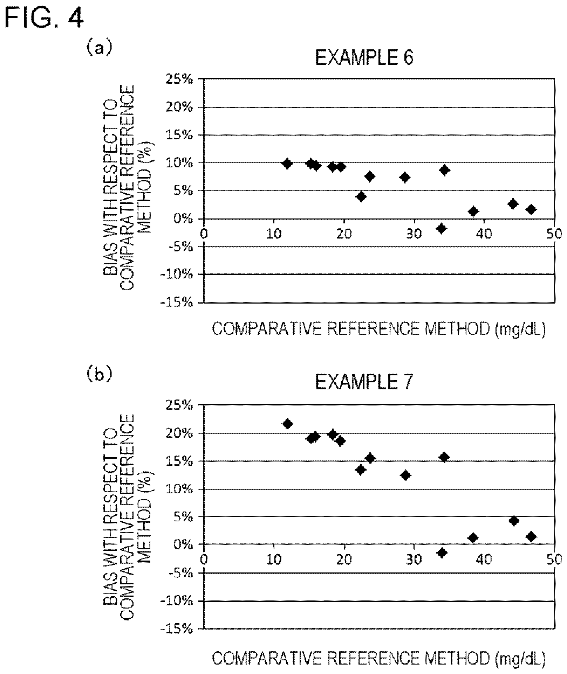

The evaluation results are shown in Figs. 1(a) to 1(c), Figs. 2(a) and 2(b), Figs. 3(a) and 3(b), and Figs. 4(a) and 4(b). In Figs. 1(a) to 1(c), Figs. 2(a) and 2(b), Figs. 3(a) and 3(b), and Figs. 4(a) and 4(b), the horizontal axis represents the measured value (concentration) of sdLDL-C by the comparative reference method, and the vertical axis represents the bias of Comparative Examples and Examples with respect to the comparative reference method. In addition, the average value of the bias is shown in Tables 1 and 3. A bias having an average value of ± 15% or less is regarded as acceptable.

From Figs. 1(a) to 1(c), Figs. 2(a) and 2(b), Figs. 3(a) and 3(b), Figs. 4(a) and 4(b), and Tables 1 and 3, it was possible to obtain sdLDL-C with high accuracy in each of Examples.

### [Table 1]

**Table 1**

| | Comparative Example 1 | Example 1 | Example 2 | Example 3 | Comparative Example 2 |
|---|---|---|---|---|---|
| Contact angle (°) | 80.1 | 74.0 | 69.8 | 66.4 | 63.5 |
| Average bias value (%) | 18.7 | 1.1 | 0.1 | 0.6 | 37.8 |

### [Table 2]

**Table 2**

| | Example 1 | Example 2 | Example 3 |
|---|---|---|---|
| Viscosity at 5°C (mPa ·s) | 1.71 | 1.76 | 1.96 |
| Viscosity at 37°C (mPa ·s) | 0.83 | 0.82 | 0.87 |

### [Table 3]

**Table 3**

| | Example 4 | Example 5 | Example 6 | Example 7 |
|---|---|---|---|---|
| Viscosity at 5°C (mPa ·s) | 1.53 | 1.72 | 1.94 | 2.97 |
| Viscosity at 37°C (mPa ·s) | 0.53 | 0.61 | 0.63 | 0.87 |
| Contact angle (°) | 70.0 | 73.6 | 72.4 | 74.2 |
| Average bias value (%) | -1.0 | -5.9 | 6.1 | 12.4 |

## Claims

1. A reagent composition comprising:
an anionic surfactant,
wherein the reagent composition is used as a first reagent composition in a method for quantifying a small dense LDL cholesterol (sdLDL-C) in a sample, the method including,
acting the first reagent composition on the sample, and
after the acting the first reagent composition on the sample, acting a second reagent composition for quantifying the sdLDL-C to quantify cholesterol in a remaining lipoprotein,
the reagent composition has cholesterol esterase activity and cholesterol oxidase activity, and also has at least one activity selected from the group consisting of peroxidase activity and catalase activity, and
a contact angle between the reagent composition and a polyethylene terephthalate (PET) base material, which is measured by the following method 1, is equal to or more than 65° and equal to or more than 77°,
(method 1)
(1) the PET base material: PET (amorphous polyester) resin plate, transparent, thickness of 2 mm,
(2) a pre-treatment: a surface of the PET base material unused is wiped with a 70% ethanol aqueous solution, and a measurement is performed within 5 minutes after the wiping,
(3) a measurement method and conditions: a liquid droplet method, a Θ/2 method, a temperature: 15 to 25°C, a syringe: Teflon-coated 18G, a dropwise addition method, a dropwise addition amount: 2 µL, a measurement 1 second after the dropwise addition, and
(4) a calculation of the contact angle: the measurement is performed 5 times, and an average value of the 5 times is obtained.

2. The reagent composition according to Claim 1,
wherein the reagent composition further has ascorbic acid oxidase activity.

3. The reagent composition according to Claim 1 or 2,
wherein the reagent composition has no sphingomyelinase activity.

4. The reagent composition according to any one of Claims 1 to 3,
wherein the anionic surfactant includes a sulfuric acid ester salt.

5. The reagent composition according to any one of Claims 1 to 4, further comprising:
any one of a hydrogen donor and a coupler.

6. The reagent composition according to any one of Claims 1 to 5,
wherein a viscosity of the reagent composition at 5°C, which is measured by the following method 2-1, is equal to or less than 2.0 mPa-s,
(method 2-1)
(1) a device: an Electro Magnetically Spinning (EMS) viscometer,
(2) a measurement method and conditions: a measurement type: an electromagnetic spinning method, a motor rotation speed: 1,000 rpm, a holding time: 600 seconds, a measurement temperature: 5°C, a sequence loop: 1 time, a sample: 500 µL, and
(3) a calculation of the viscosity: the measurement is performed 5 times, and an average value of the 5 times is calculated.

7. The reagent composition according to any one of Claims 1 to 6,
wherein a viscosity of the reagent composition at 37°C, which is measured by the following method 2-2, is equal to or less than 0.89 mPa·s,
(method 2-2)
(1) a device: an Electro Magnetically Spinning (EMS) viscometer,
(2) a measurement method and conditions: a measurement type: an electromagnetic spinning method, a motor rotation speed: 1,000 rpm, a holding time: 600 seconds, a measurement temperature: 37°C, a sequence loop: 1 time, a sample: 500 µL, and
(3) a calculation of the viscosity: the measurement is performed 5 times, and an average value of the 5 times is calculated.

8. A kit used for quantifying a sdLDL-C in a sample, the kit comprising:
a first reagent composition consisting of the reagent composition according to any one of Claims 1 to 7; and
a second reagent composition for quantifying the sdLDL-C.

9. The kit according to Claim 8,
wherein the second reagent composition has peroxidase activity.

10. The kit according to Claim 8 or 9,
wherein the first reagent composition contains any one of a hydrogen donor and a coupler and does not contain the other, and
the second reagent composition does not contain the one of the hydrogen donor and the coupler and contains the other.
